Europäisches Patentamt

European Patent Office

Office européen des brevets

(19)

(11) Publication number: **0 157 071**
**B1**

(12)

# EUROPEAN PATENT SPECIFICATION

(45) Date of publication of patent specification: 26.07.89

(21) Application number: 84850107.8

(22) Date of filing: 03.04.84

(51) Int. Cl.⁴: **G 01 N 33/52,**
G 01 N 33/558, C 12 Q 1/18,
G 01 N 31/22

(54) Method and device for producing varying concentration patterns of chemically or biologically active substances.

(43) Date of publication of application:
09.10.85 Bulletin 85/41

(45) Publication of the grant of the patent:
26.07.89 Bulletin 89/30

(84) Designated Contracting States:
AT BE CH DE FR GB IT LI NL SE

(56) References cited:
WO-A-82/02251
FR-A-2 264 089
US-A-3 510 263
US-A-3 932 223

(73) Proprietor: AB BIODISK
Pyramidvägen 7
S-171 36 Solna (SE)

(72) Inventor: Ericsson, Magnus
Riddargatan 70
S-114 57 Stockholm (SE)
Inventor: Bolmström, Anne
Kanalvägen 5
S-184 00 Akersberga (SE)

(74) Representative: Onn, Thorsten et al
AB STOCKHOLMS PATENTBYRA Box 3129
S-103 62 Stockholm (SE)

The file contains technical information submitted after the application was filed and not included in this specification

Courier Press, Leamington Spa, England.

## Description

This invention relates to a method and a device for producing varying concentration patterns of chemically or biologically active substances.

The assay of chemically or biologically active substances in body fluids, for example blood, liquor, pus, sputum, secretion, bladder content or urine, is very essential in monitoring therapeutic treatment with such substances. With such a treatment, it is of critical importance to achieve therapeutic concentrations of the substance in question and to prevent a toxic concentration from being exceeded. Further, there is a great demand for the quantification of chemically or biologically active substances in various biologic materials, for example soil, cell cultures, allergen extracts and other cell extracts.

One of the methods of determining chemically or biologically active substances quantitatively in body fluids is the microbiologic method, i.e. it is based on the capacity of an active substance to promote or to inhibit growth of a suitable indicator microorganism. A well known method in this respect is the so-called paper disc method, which has been described a.o. by Jalling et al., Europ. J. Clin. Pharmacol. 4,150—157 (1972). This method is based on a number of paper discs being impregnated with different amounts, representing different concentrations of a reference substance, for example an antibiotic. The sample containing the same active substance, which is to be assayed, is also impregnated onto a paper disc of the same kind. All of these paper discs, references with defined, varying concentrations, and samples with unknown concentrations are applied onto the surface of a culture medium, which has been inoculated with a selected microorganism. Distinct inhibition zones are formed around the reference discs as well as the discs impregnated with samples. The diameters of the inhibition zones around the reference discs are plotted against the concentrations to form a standard curve. The concentration corresponding to the zone diameter of the sample is then interpolated from the plotted standard curve. This method involves certain drawbacks such as a high number of discs to be tested on the same test plate, and that a new standard curve must be established on each test occasion.

Concentrations of enzymes and antigens are determined in an analogous manner using immunodiffusion methods, which have been described a.o. by Ouchterlony, Ö. & Nilsson, L.-Å.: Immunodiffusion and immunoelectrophoresis, I: Handbook of Experimental Immunology. Ed. D. M. Weir, Blackwell, Oxford, 2nd ed. (1973) and Mancini, G., Carbonara, A. O. & Heremans, J. F.: Immunochemical quantitation of antigens by single radial immunodiffusion, Immunochemistry 2 (1965) p. 235. In these techniques, the diffusion layer may consist, for example, of a thin layer of agargel containing a substrate specific for the enzyme to be determined or an antiserum or antibodies specific for the antigen to be determined. The enzyme or antigen is applied to the substrate in round holes, i.e. solutions are filled into wells made in the agar layer. The enzyme or antigen diffuses out radially from the diffusion centre, i.e. the well containing the solution. The reference wells containing defined varying concentrations of the enzyme or antigen, and the well containing the sample with an unknown concentration are tested on the same agargel plate.

The circular zone around an enzyme well can consist of a transparency in the otherwise turbid gel layer, for example in the assay of protease concentration in an agargel containing casein. The circular zone around an antigen well can consist of a precipitate in the otherwise clear gel layer. Reading and interpretation of results are similar to those described for the microbiologic test. The diameter (or area) of the zone around the unknown sample is read against a standard curve based on the diameters (or areas) around the reference wells.

Another immunodiffusion technique used for determining antigens or antibodies is "double diffusion (Ouchterlony) precipitation". Antigen as well as antibodies are filled in wells and allowed to diffuse toward each other. Where these substances meet in optimal proportions in the gel, a precipitate line will appear. The relative position of the line is related to the absolute concentration of the antigen or antibody in the respective wells, and its molecule size and rate of diffusion through the gel.

These methods have similar drawbacks as those of the microbiologic test. Moreover, these tests are time consuming since the large molecules involved diffuse slowly. The point of stationary equivalence is established after a long period of diffusion. The accuracy of results will be affected by the instability of concentration gradients unless readings are made at suitable times.

The sensitivity of microorganisms, for example bacteria, towards a biologically or chemically active substance is at present determined by measuring the minimum inhibitory concentration (MIC). MIC determinations by available methods are complicated, time consuming and expensive. Two different methods are essentially used today for determining MIC values. One of the methods comprises a series of two-fold dilutions of the active substance in a fluid or solid medium. This method has the disadvantage of giving less precise and less accurate information on the sensitivity of a microorganism because it is based on a discontinuous set of dilutions. The other method is based on the diffusion of the active substance from a defined diffusion centre in an agar layer. The concentration gradients of different substances depend on the particular diffusion properties of the individual substances. Therefore for each substance a regression line correlating the logarithm of MIC values (determined by the dilution method) to inhibition zone diameters around a disc containing a defined amount of the substance, must be established for different group of bacteria (Ericsson, H. M. & Sherris, J. C., Antibiotic Sensitivity Testing, Acta Path. & Microbiol. Scand., Section B 1971, Supplement No. 217).

Another method for investigating microorganisms, i.a. determining the minimum inhibitory concentration, is disclosed in FR—A—2 264 089. In one embodiment described therein the biologically active substance is supplied to the cultivating medium along an elongated zone in an amount varying continuously in predetermined manner through out the length of the zone. The active substance is applied by the aid of a porous strip, on which it has been impregnated. One active substance can be impregnated per strip and that in a linear concentration gradient. The absorption ability of the strip can be improved by incorporating agar therein and the strip may vary in area, porosity and shape from one end to the other as a means of generating the linear concentration gradient.

The use of a porous strip is also disclosed in US—A—3 510 263 as test papers for carrying out quantitative chemical analyses by titrametric methods. The test papers disclosed in this patent include a bibulous member that is impregnated with a predetermined titrant in a continuous zone of linearly increasing concentration. The paper is also impregnated with an indicator suitable for signalling the equivalence point of the titrant reaction product involved in the analysis. The test paper is dipped into test liquid so that the portion of the bibulous member having the impregnated titration zone becomes saturated. The test paper is then removed and the colour change on development is being observed. The presence of the indicator will cause a colour change all along the zone until the concentration of titrant is such that the equivalence point is reached.

The disadvantages of the afore-mentioned methods can be eliminated by the present invention whereby predetermined and defined varying concentration patterns of chemically or biologically active substances in a medium to be used in qualitative or quantitative determinative methods are attained by contacting a thin, inert carrier made of non-porous material having a rectangular area on which the active substances are present in the desired, predetermined concentration pattern, with the medium containing chemical or biological material reacting with said substances, whereby imprints of the desired, predetermined concentration pattern are created within the entire rectangular area or in a portion thereof on, under or integrated with the surface of the medium, and that the predefined concentration pattern on the carrier have at least one concentration maximum and one concentration minimum, and that at least one of these is preferably located adjacent to one end of the rectangular area.

According to the invention, the medium can be a culture medium, e.g. a layer of agar or some other polymer containing suitable growth factors when required, or the medium may be a diffusion layer and/or a reaction layer.

The chemical or biologic material in the medium can be, for example microorganisms, other biologic cells, antiserum, antibodies or enzyme substrates.

The biologically or chemically active substances are, for example, antimicrobial substances, microbiologically active substances, substances having an inhibitory, lethal, toxic or mutagenic effect on cells.

The substance or substances can be applied so that the concentrations thereof in the rectangular area and respectively on the carrier is highest in the centre and decreases towards the two ends, or that the concentration is highest at one end of the rectangular area and decreases towards the other end. Further, the concentration of a first substance can be uniformly distributed over the rectangular area, and respectively carrier, while a second substance is present with a concentration gradient decreasing from one end to the other. A first substance may also have a concentration gradient in the rectangular area, and respectively carrier, decreasing from one end to the other, while a second substance is applied with a concentration gradient decreasing in a direction opposite to the gradient of the first substance. The substance or substances may also be applied in the rectangular area, and respectively on the carrier, in such a way that several concentration maxima and minima are found in the aforesaid area and respectively carrier.

A substance also may be applied on a portion of the rectangular area, and respectively carrier, with the concentration decreasing from one end of the rectangle to the other, and in the portion of the area and respectively carrier, free from substance, a sample the concentration of which is to be determined, is applied.

A specific concentration pattern in a medium may be attained, for example by applying a rectangular carrier, on which the substance or substances prevail in the desired concentration pattern, to the surface of the medium or vice-versa. A specific concentration pattern can also be attained by applying a number of units, which contain the substance or substances in known concentrations, to the surface of a medium in such a way, that the specific concentration pattern is obtained in the rectangular area.

Examples of carriers suitable for use in the present invention are thin sheets of neutral, non-porous material, for example polyacrylamide, polyester, polyamide or similar material. These materials can be opaque or transparent.

The culture medium can also be applied in the form of a thin layer of a dehydrated medium on the carrier, for example of the type described in the international patent application PCT/US 82/00085 (publication No. WO 82/02563).

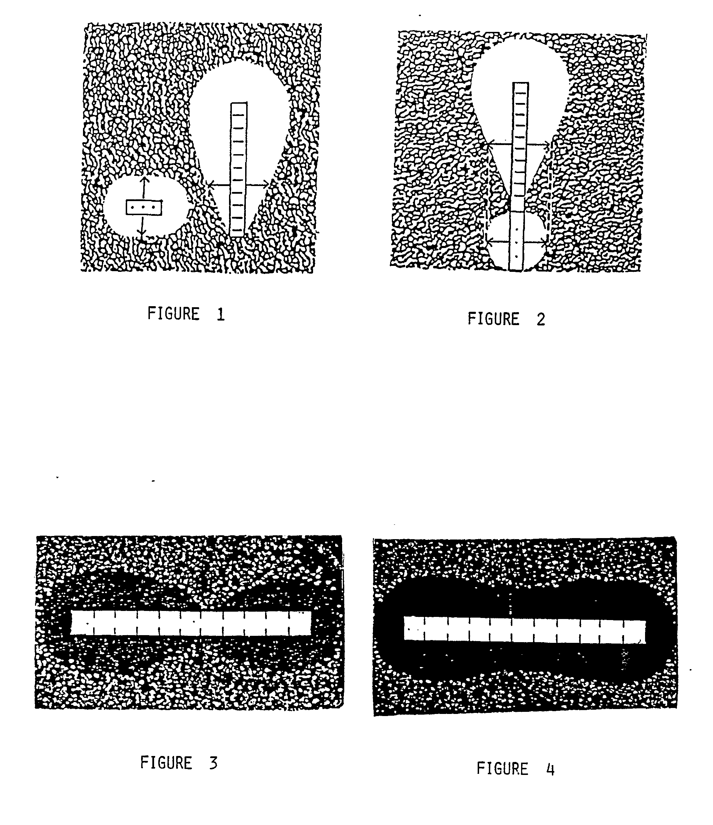

In one embodiment of a device according to the present invention, the substance is applied on the entire surface of the carrier, with a concentration gradient decreasing from a maximum at one end of the carrier to a minimum at the other end. The substance can be applied in a manner known per se, for example by micropipetting specific volumes of reference solutions having defined decreasing concentrations onto the surface of the carrier, at certain intervals from each other, resulting in a continuous concentration gradient along the carrier. When the concentration of a substance in a body fluid is to be determined for example, a sample thereof can be pipetted onto a unit, which is then applied to the surface of a culture medium which has been inoculated with a selected microorganism. the rectangular carrier with a concentration gradient of the reference substance is simultaneously applied to the same test plate, which is then incubated in the usual manner. Along the rectangular carrier a drop-shaped inhibition zone is formed where growth of the microorganism is inhibited. The width of the zone is largest at the end of the carrier where the highest concentration of the reference substance is to be found and tapers accordingly along the decreasing concentration gradient towards the end of the carrier where the lowest concentration of the reference substance is located. An inhibition zone is also formed around the unit containing the sample. This is illustrated in Figure 1.

The width of the zone around the sample unit is measured and compared to the width of the drop-shaped zone along the concentration gradient of the reference, whereby the unknown concentration can be interpolated as indicated by arrows in Figure 1. To facilitate a direct reading of the concentration, the rectangular carrier is preferably graded with a concentration scale.

In another embodiment of a device according to the present invention, the reference substance is applied on a portion of the carrier with a concentration gradient decreasing from a maximum at one end of the carrier. A sample of the body fluid containing the substance, the concentration of which is to be determined, is applied by means of a micropipette onto the portion of the rectangular carrier free from reference substance. The carrier is preferably graded with a scale for direct reading of the concentration. The carrier after application of the unknown sample, is applied onto the surface of a culture medium, previously inoculated with a selected microorganism. The test is then carried out in the usual manner. A drop-shaped inhibition zone is formed where no growth of microorganism occurs around the rectangular carrier, and an inhibition zone is formed around the carrier where the test sample has been applied (see Figure 2). This form of the device allows a direct reading of the concentration in the sample, by a comparison of the width of the zone around the sample to the width of the drop-shaped zone along the concentration gradient of the reference.

These two embodiments can be used in an analogous way for the assay of enzymes, antigens and growth promoting substances, such as vitamins, where the drop-shaped zone along the concentration gradient of the reference and the zone around the sample may consist of a transparency or precipitate or growth of microorganisms in contrast to inhibition of growth.

In a biochemical analysis, for example of the nitrate content in soil using the enzyme nitrate reductase, the reference substrate is applied on a portion of the carrier surface with a concentration gradient decreasing from a maximum at one end of the carrier. A sample of the soil containing nitrate, the concentration of which is to be determined, is applied by means of a micropipette onto the portion of the rectangular carrier free from the reference substance. The carrier is preferably graded with a scale for a direct reading of the concentration. A defined amount of the specific enzyme which reacts with the nitrate is applied uniformly on the entire surface of the carrier Suitable reagents, for example sulphanilic acid and N,N-dimethyl-1-naphthylamine, which can show this enzyme-substrate reaction in the form of a colour change are applied onto the entire carrier surface, or is present as an integrated part of the carrier. The intensity of the colour of the sample is compared to the range of varying colour intensities along the calibrated portion of the carrier consisting of a concentration gradient of the reference substrate.

These two aforementioned embodiments can preferably be used a.o. for the determination of unknown concentrations of active substances in different samples.

In yet another embodiment of the device according to the present invention, a first substance is applied in a concentration uniformly distributed over the rectangular carrier, while a second substance is applied in a concentration gradient decreasing from one end to the other end of the carrier.

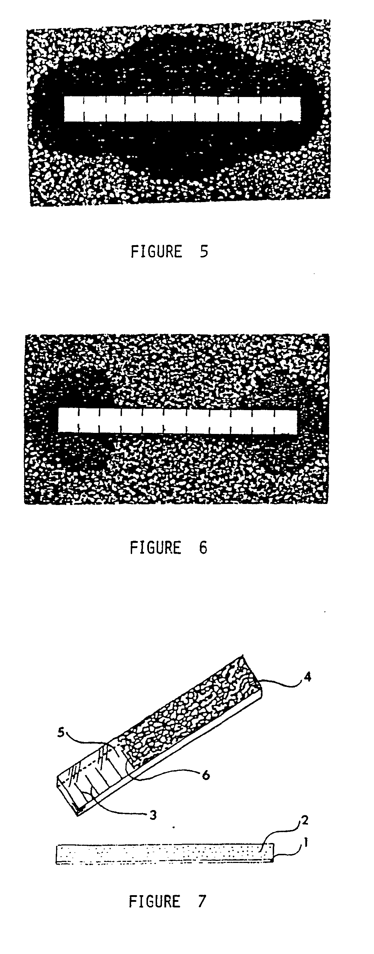

In a further embodiment of the device according to the invention, a first substance is applied in a concentration gradient decreasing from one end of the carrier to the other, while a second substance is applied in a concentration gradient which decreases in a direction opposite to the gradient of the first substance. The concentration of the first substance is thus highest where the concentration of the second substance is lowest, and vice-versa. This embodiment of the device can be used to study the interaction between different substances, for example the binding of antimicrobial substances to proteins, the combined action of antibiotics resulting in additative effects, synergism or antagonism. Figures 3—6 illustrate the interaction between two antibiotics A and B, which are applied on the same carrier, the concentration gradients of which decrease in opposite directions as described above. After application of the device on an inoculated medium, and after incubation inhibition zones of varying shapes will be obtained. In Figure 3, two drop-shaped zones on opposite ends indicate that substances A and B act independently of each other. In Figure 4, the ends of the otherwise drop-shaped zones instead of tapering towards the edge of the carrier, merge into a common inhibition zone, indicating an additative effect. In Figure 5, the ends of the otherwise drop-shaped zones merge into a common inhibition zone which is larger than the zones at either end of the carrier, indicating a synergistic effect. In Figure 6, the edges of the otherwise drop-shaped zones abruptly transect the carrier edge, instead of gradually tapering towards it, indicating an antagonistic effect.

The aforementioned embodiments are suitable for use, for example in determining the concentration ranges and proportions between two or more substances interacting in an additative, synergistic or antagonistic manner. This invention provides a simple means of determining the optimal ratios of two or more substances to be administered together in guiding the therapy with a combination of drugs, e.g. antibiotics, antimycotics and cytostatic drugs.

Figure 7 shows a schematic illustration of one embodiment of a device according to the invention comprising a transparent or opaque carrier 1, with a printed scale 3 indicating the concentration of the active substance or other related parameters thereof. On the carrier, a predetermined concentration pattern of an active substance has been applied. 2 is a growth medium and/or diffusion layer and/or other reaction layer that has been inoculated with a microorganism or other biological or biochemical indicator which interacts with the active substance on the carrier. 4 is the zone of growth of the microorganism or other visible biological or biochemical reaction. 5 is the zone of inhibition or growth of a microorganism or zone of other reaction/no reaction for other indicator systems. 6 is the border between the zones of growth/inhibition for a microorganism or other visible reaction/no reaction or other two discriminatory reactions for other indicator systems. The concentration of the active substance or other parameters related thereof which is associated with the inhibition of growth, i.e. MIC (minimum inhibitory concentration) or other characteristic reaction, can be easily read directly on the device.

For the determination of the minimum inhibitory concentration (MIC) and the minimum bactericidal concentration (MBC), the latter of which is the lowest concentration of an antimicrobial substance inhibiting visible growth of microorganisms in an irreversible manner, i.e. killing of the inoculum under a set of defined test conditions, a rectangular carrier of the kinds earlier described can be used, whereby the MIC value or related parameter thereof can be read directly as the growth/inhibition border (6 in Figure 7) on the carrier or as the point where the tapering border of the drop-shaped zone along the carrier transects the edge of the carrier. The MBC value can be subsequently determined by replacing the carrier containing the antimicrobial substance, around which a drop-shaped zone has been formed, with a new carrier containing suitable inactivating substance, for example enzymes or other chemicals which can effectively inactivate the residual antimicrobial substance in the culture medium. After additional incubation in an antimicrobial free medium the size and shape of the drop-shaped zone, or the position of the growth/inhibition border on the carrier as in Figure 7, will change in relation to the MBC value.

The method and the device according to the present invention, can thus be used, for example to characterize the sensitivity or other property of microorganisms and other biological cells to antimicrobial substances, and respectively to substances which may have an inhibitory, lethal, toxic, mutagenic or growth promoting effect on the cells. The method and device can also be used to quantify biologically or biochemically active substances and substrate-specific enzymes or enzyme-specific substrates, to assess the cancerogenic, mutagenic or toxic effects of different substances and to select mutants of microorganisms or other biological cells resistant to antimicrobial agents or other drugs or dependent on different growth factors.

The term "microorganism" refer to bacteria, such as the Enterobactiaceae, *Staphylococci, Streptococci, Hemofilus,* Neisseriaceae, *Bacteroides,* and *Clostridia, Mycobacteria, Actinomyces, Mycoplasma, Nocardia,* virus, and fungi such as molds, yeasts and *Candida.*

The term "biological cells" comprises cancer cells, normal human cells, animal cells and plant cells, of the type "stem cells".

Antimicrobial substances are antibiotics, for example aminoglycosides, $\beta$-lactam antibiotics, macrolide antibiotics, polymyxins, polypeptides and other chemotherapeutics such as sulfonamides, antimycotics, for example 5-fluorocytosine, amphotericin, antiviral agents such as adenine arabinoside (Ara-A), trifluorothymidine, anti-tuberculous drugs, such as isoniazide and cycloserine, and disinfectants, antiseptics and preservatives such as chlorohexidine, ethanol and benzalkonium chloride.

Substances which may have an inhibitory, lethal, toxic or mutagenic effect on cells are anticancer agents, for example anthracyclines, mitomycins, cancerogenic/mutagenic substances such as dimethylnitrosamine, herbicides such as paraquat and pesticides and insecticides such as dicophane (DDT).

Microbiologically active substances, are in addition to the aforementioned antimicrobial substances, bacteriocins, for example colicins, different growth factors, such as amino acids, vitamins and other nutrients, different body fluids and components thereof such as serum, blood and specific components in serum, for example $\beta$-lysine.

Biologically active substances are interferons, such as human interferon HuIFN-$\beta$, IF4N-$\beta$, enzymes such as $\beta$-lactamases, proteases and ureases, enzyme specific substrates such as nitrates, $\beta$-lactam antibiotics, aminoglycosides and chloramphenicol, antigens such as immunoglobulins, polysaccharides and toxins from microorganisms.

## Claims

1. A method for producing predetermined and defined varying concentration patterns of chemically or biologically active substances in a medium to be used in qualitative or quantitative determinative methods, characterized in that the concentration patterns are attained by contacting a thin, inert carrier made of non-porous material having a rectangular area on which the active substances are present in the desired predetermined concentration pattern, with the medium containing chemical or biological material reacting with said substances, whereby imprints of the desired predetermined concentration pattern are created within the entire rectangular area or in a portion thereof on, under or integrated with the surface of the medium, and that the predefined concentration pattern on the carrier have at least one concentration maximum and one concentration minimum, and that at least one of these is preferably located adjacent to one end of the rectangular area.

2. The method of claim 1, characterized in that the medium is a culture medium or diffusion layer or other reaction layer and that the chemical or biological material is a microorganism or other biological cell, an enzyme substrate, an enzyme, a chemical indicator, an antiserum, an antibody or an antigen.

3. The method of any of claims 1—2, characterized in that at least one substance is applied so that the concentration thereof is greatest in the centre of the rectangular area and decreases towards both ends of the pattern.

4. The method of any of the claims 1—2, characterized in that at least one substance is applied so that the concentration thereof decreases from one end of the rectangular area to the other.

5. The method of any one of the claims 1—2, characterized in that a first substance is applied in a concentration uniformly distributed over the entire rectangular area, and a second substance is applied in a concentration gradient decreasing from one end of the rectangular area to the other.

6. The method of any one of claims 1—2, characterized in that a first substance is applied in a concentration gradient decreasing from one end of the rectangular area to the other end, and a second substance is applied in a concentration gradient decreasing in a direction opposite to the gradient of the first substance.

7. A device for producing predetermined and defined varying concentration patterns of chemically or biologically active substances in a medium to be used in qualitative or quantitative determinations, characterized in that it comprises a rectangular thin, inert carrier made of a non-porous material on which the active substances are present in the desired, predetermined concentration pattern, and on which one or more substances are applied on the entire carrier or on a portion thereof, in such a manner, that they are present in a concentration pattern having at least one concentration maximum and one concentration minimum, that at least one of these is preferably located adjacent to one of the ends of the carrier, and that said carrier when in contact with the reaction medium creates imprints of the concentration pattern with the entire rectangular area or in a portion thereof on, under or integrated with the surface of the medium.

8. The device of claim 7, characterized in that at least one substance is applied so that the concentration thereof is greatest in the centre of the carrier and decreases towards both ends of the carrier.

9. The device of claim 7, characterized in that at least one substance is applied so that the concentration thereof decreases from one end of the carrier to the other end.

10. The device of claim 7, characterized in that a reference substance is applied on a portion of the carrier with a concentration gradient decreasing from a maximum at one end of the carrier, and that on the portion of the carrier free from reference substance, a sample containing the same substance, the concentration of which is to be determined, is applied.

11. The device of claim 7, characterized in that a first substance is applied in a concentration uniformly distributed over the rectangular carrier, and a second substance is applied in a concentration gradient decreasing from one end of the carrier to the other end thereof.

12. The device of claim 7, characterized in that a first substance is applied in a concentration gradient decreasing from one end of the carrier to the other end thereof, and a second substance is applied in a concentration gradient decreasing in a direction opposite to the gradient of the first substance.

13. The device of any one of the claims 7—12, characterized in that the carrier consists of a culture medium and/or a diffusion layer and/or a reaction layer, and/or an inert carrier material.

## Patentansprüche

1. Herstellungsverfahren für im Voraus bestimmte und definierte sich ändernde Konzentrationsmuster chemisch oder biologisch aktiver Substanzen in einem Medium zur Verwendung in qualitativer oder quantitativer Analyse, dadurch gekennzeichnet, dass die Konzentrationmuster dadurch erstellt werden, dass ein dünner inerter Träger aus nicht porösem Material mit einer Rechteckfläche, auf der die aktiven Substanzen im gewünschten, im Voraus bestimmten und definierten Konzentrationsmuster vorliegen, mit dem das chemisch oder biologisch aktive Material enthaltende, mit der Substanz reagierenden, Medium in Kontakt kommen, wodurch Abdrücke des gewünschten, im Voraus bestimmten Konzentrationsmusters innerhalb der gesamten Rechteckfläche oder einem Teil derselben unter oder integriert mit der Oberfläche des Mediums zustande kommen, und dass das im Voraus bestimmte Konzentrationsmuster auf dem Träger mindestens eine Konzentrationsspitze und eine Konzentrationssenke aufweist, und dass mindestens eine derselben vorzugsweise neben einem Ende der Rechteckfläche zu liegen kommt.

2. Verfahren gemäss Patentanspruch 1, dadurch gekennzeichnet, dass das Medium eine Kultur oder ein Diffusionsschicht oder eine andere Reaktionsschicht ist, und dass das chemisch oder biologisch aktive Material ein Mikroorganismus oder eine andere biologische Zelle, ein Enzymsubstrat, ein Enzym, ein chemischer Indikator, ein Antiserum, ein Antikörper oder ein Antigen ist.

3. Verfahren gemäss einem der Patentansprüche 1—2, dadurch gekennzeichnet, dass mindestens eine Substanz so verwendet wird, dass deren Konzentration in der Mitte der Rechteckfläche so gross wie möglich ist und zu beiden Enden des Musters abnimmt.

4. Verfahren gemäss einem der Patentansprüche 1—2, dadurch gekennzeichnet, dass mindestens eine Substanz so verwendet wird, dass deren Konzentration von einem Ende der Rechteckfläche zum anderen abnimmt.

5. Verfahren gemäss einem der Patentansprüche 1—2, dadurch gekennzeichnet, dass eine erste Substanz in einer Konzentration die über die gesamte Rechteckfläche einheitlich verteilt ist, und eine zweite Substanz mit einer Konzentrationsneigung, die von einem Ende der Rechteckfläche zum anderen abnimmt, verwendet wird.

6. Verfahren gemäss einem der Patentansprüche 1—2, dadurch gekennzeichnet, dass eine erste Substanz mit einer Konzentrationsneigung, die von einem Ende der Rechteckfläche zum anderen abnimmt, und eine zweite Substanz mit einer Konzentrationsneigung, die in einer Neigung in einer der ersten Substanz entgegengesetzten Richtung abnimmt, verwendet wird.

7. Anordnung für die Herstellung eines im Voraus bestimmten und definierten sich ändernden Konzentrationsmusters chemisch oder biologisch aktiver Substanzen in einem Medium zur Verwendung in qualitativer oder quantitativer Analyse, dadurch gekennzeichnet, dass die Anordnung einen rechteckigen, dünnen, inertem Träger aus nicht porösem Material umfasst, auf dem die aktive Substanz im gewünschten, im Voraus bestimmten Konzentrationsmuster vorliegt und auf dem eine oder mehrere Substanzewn über den gesamten Träger, oder einen Teil desselben, auf solche Weise angebracht werden, dass die in einem Konzentrationsmuster mit mindestens einer Konzentrationsspitze und einer Konzentrationssenke vorliegen, dass mindestens eine derselben vorzugsweise neben einem Ende der Rechteckfläche zu liegen kommt, und dass der Träger bei Kontakt mit dem Reaktionsmedium Abdrücke des gewünschten, im Voraus bestimmten Konzentrationsmusters innerhalb der gesamten Rechteckfläche oder einem Teil derselben erstellt.

8. Anordnung gemäss Patentanspruch 7, dadurch gekennzeichnet, dass mindestens eine Substanz so verwendet wird, dass deren Konzentration am grössten in der Mitte des Trägers ist und zu den Kanten des Trägers abnimmt.

9. Anordnung gemäss Patentanspruch 7, dadurch gekennzeichnet, dass mindestens eine Substanz so verwendet wird, dass deren Konzentration von einem Ende des Trägers zum anderen abnimmt.

10. Anordnung gemäss Patentanspruch 7, dadurch gekennzeichnet, dass ein Referenzsubstanz auf einem Teil des Trägers mit einer Konzentrationsneigung, die von einem Höchstwert an einem Ende des Trägers abnimmt, angebracht wird, und dass auf dem von der Referenzsubstanz freien Teil des Trägers eine die gleiche Substanz enthaltende Probe angebracht wird, deren Konzentration bestimmt werden soll.

11. Anordnung gemäss Patentanspruch 7, dadurch gekennzeichnet, dass eine erste Substanz in einer über den rechteckigen Träger verteilten einheitlichen Konzentration, sowie eine zweite Substanz mit einer von einen Ende des Trägers zum anderen abnehmenden Konzentrationsneigung angebracht wird.

12. Anordnung gemäss Patentanspruch 7, dadurch gekennzeichnet, dass eine erste Substanz mit einer vom einen Ende des Trägers zum anderen Ende abnehmenden Konzentrationsneigung, sowie eine zweite Substanz mit einer Konzentrationsneigung die der Richtung der Neigung der ersten Substanz entgegengesetzt ist, angebracht wird.

13. Anordnung gemäss einem der Patentansprüche 7—12, dadurch gekennzeichnet, dass der Träger aus einer Kultur, und/oder einer Diffusionsschicht und/oder einer Reaktionsschicht und/oder einem inerten Trägermaterial besteht.

## Revendications

1. Méthode pour produire des modèles prédéterminés et définis de concentrations variables de substances chimiquement ou biologiquement actives dans un milieu à utiliser dans des méthodes de détermination qualitative ou quantitative, caractérisé en ce que l'on obtient lesdits modèles de concentrations en mettant un support inerte mince, réalisé en une matière sans porosités et comportant une zone rectangulaire dans laquelle les substances actives sont présentes selon le modèle prédéterminé de concentrations souhaité, en contact avec le milieu contenant de la matière chimique ou biologique réagissant avec lesdites substances, créant ainsi des empreintes du modèle prédéterminé de concentrations souhaité dans la totalité de ladite zone rectangulaire ou dans une partie de celle-ci, sur, sous ou dans la surface dudit milieu, que le modèle prédéfini de concentrations obtenu sur le support présente au moins un maximum et un minimum de concentration, et que l'un d'eux au moins est de préférence voisin de l'une des extrémités de ladite zone rectangulaire.

2. Méthode selon la revendication 1, caractérisée en ce que ledit milieu est un bouillon de culture ou une couche de diffusion ou autre couche réactive, et que ladite matière chimique ou biologique est un microorganisme ou autre cellule biologique, un substrat enzymatique, une enzyme, un indicateur chimique, un antisérum, un anticorps ou un antigène.

3. Méthode selon l'une des revendications 1 et 2, caractérisée en ce qu'une substance au moins est appliquée de manière que sa concentration soit au maximum au centre de ladite zone rectangulaire pour aller décroissant vers les deux extrémités du modèle.

4. Méthode selon l'une des revendications 1 et 2, caractérisée en ce qu'une substance au moins est appliquée de manière que sa concentration aille décroissant d'une extrémité de la zone rectangulaire à l'autre.

5. Méthode selon l'une des revendications 1 et 2, caractérisée en ce qu'une première substance est appliquée dans une concentration également répartie sur toute la zone rectangulaire, et qu'une seconde substance est appliquée avec un gradient de concentration qui va décroissant d'une extrémité à l'autre de la zone rectangulaire.

6. Méthode selon l'une des revendications 1 et 2, caractérisée en ce qu'une première substance est appliquée avec un gradient de concentration qui va décroissant d'une extrémité à l'autre de la zone rectangulaire, et qu'une seconde substance est appliquée avec un gradient de concentration qui va décroissant dans un sens opposé à celui du gradient de ladite première substance.

7. Dispositif pour produire des modèles prédéterminés et définis de concentrations variables de substances chimiquement ou biologiquement actives dans un milieu à utiliser pour des déterminations qualitatives ou quantitatives, caractérisé en ce qu'il comprend un support inerte mince rectangulaire, réalisé en une matière sans porosités, sur lequel les substances actives sont présentes selon le modèle prédéterminé de concentrations souhaité, et sur lequel une ou plusieurs substances sont appliquées, sur la totalité ou sur une partie dudit support, de manière qu'elles soient présentes selon un modèle de concentrations comportant au moins un maximum et un minimum de concentration, que l'un de ceux-ci au moins est de préférence voisin de l'une des extrémités du support, et que ledit support, mis en contact avec le milieu réactif, crée des empreintes du modèle de concentrations dans la totalité ou dans une partie de la zone rectangulaire, sur, sous ou dans la surface dudit milieu.

8. Dispositif selon la revendication 7, caractérisé en ce qu'une substance au moins est appliquée de manière que sa concentration soit au maximum au centre dudit support pour aller décroissant vers les deux extrémités dudit support.

9. Dispositif selon la revendication 7, caractérisé en ce qu'une substance au moins est appliquée de manière que sa concentration aille décroissant d'une extrémité à l'autre dudit support.

10. Dispositif selon la revendication 7, caractérisé en ce qu'une substance de référence est appliquée sur une partie du support, avec un gradient de concentration qui va décroissant à partir d'un maximum situé à l'une des extrémités du support, et que, sur la partie du support exempte de substance de référence, un échantillon contenant cette même substance, dans une concentration à déterminer, est appliqué.

11. Dispositif selon la revendication 7, caractérisé en ce qu'une première substance est appliquée dans une concentration également répartie sur tout le support rectangulaire, et qu'une seconde substance est appliquée avec un gradient de concentration qui va décroissant d'une extrémité à l'autre dudit support.

12. Dispositif selon la revendication 7, caractérisé en ce qu'une première substance est appliquée avec un gradient de concentration qui va décroissant d'une extrémité à l'autre dudit support, et qu'une seconde substance est appliquée avec un gradient de concentration qui va décroissant dans un sens opposé à celui du gradient de ladite première substance.

13. Dispositif selon une quelconque des revendications 7 à 12, caractérisé en ce que ledit support est constitué par un bouillon de culture et/ou une couche de diffusion et/ou une couche réactive et/ou une matière inerte de support.

FIGURE 1

FIGURE 2

FIGURE 3

FIGURE 4

FIGURE 5

FIGURE 6

FIGURE 7